# FASCICULE DE BREVET EUROPEEN

(11) **EP 1 246 690 B1**
(45) Date de publication et mention de la délivrance du brevet: **17.12.2003**
(21) Numéro de dépôt: 01903930.4
(22) Date de dépôt: 12.01.2001
(51) Int. Cl.: B01F 17/00

(54) **NOUVELLE FAMILLE DE COMPOSITIONS A BASE D'ALKYLPOLYGLYCOSIDES ET DE DIOLS GRAS, NOTAMMENT UTILES POUR LA PREPARATION D'EMULSIONS STABLES ET PRESENTANT UN BON EFFET OPACIFIANT**
EINE NEUE FAMILIE VON ZUSAMMENSTELLUNGEN AUS ALKYLPOLYGLYKOSIDEN UND FETTDIOLEN, INSBESONDERE VERWENDBAR FÜR DIE BEREITUNG VON STABILEN EMULSIONEN MIT EINEM GUTEN TRÜBUNGSEFFEKT
NOVEL FAMILY OF ALKYLPOLYGLYCOSIDE AND FATTY DIOL BASED COMPOSITIONS, WHICH ARE PARTICULARLY SUITABLE FOR PREPARING STABLE EMULSIONS AND HAVE A GOOD OPACIFYING EFFECT

(30) Priorité: 12.01.2000 FR 0000319
(43) Date de publication de la demande: 09.10.2002
(73) Titulaire: SOCIETE D'EXPLOITATION DE PRODUITS POUR LES INDUSTRIES CHIMIQUES, S.E.P.P.I.C., 75321 Paris Cédex 07 (FR); Milius, Alain, 06000 Nice (FR); Boiteux, Jean-Pierre, 81710 Saix (FR); Rolland, Hervé, 81100 Castres (FR); Tabacchi, Guy, 75007 Paris (FR); Amalric, Chantal, 81700 Blan (FR)
(72) Inventeur: MILIUS, Alain, F-06000 Nice (FR); BOITEUX, Jean-Pierre, F-81710 Saix (FR); ROLLAND, Hervé, F-81100 Castres (FR); TABACCHI, Guy, F-75007 Paris (FR); AMALRIC, Chantal, F-81700 Blan (FR)
(74) Mandataire: Hubert, Philippe
(86) Numéro de dépôt international: FR0100094
(87) Numéro de publication internationale: WO01051191

(56) Documents cités:
- Aucun document pertinent relevé

## Description

La présente invention concerne une nouvelle famille de compositions à base d'alkylpolyglycosides et de diols gras, notamment utiles pour la préparation d'émulsions stables et présentant un bon effet opacifiant.

L'invention trouve notamment application dans le domaine cosmétique, en particulier pour la préparation de formulations moussantes.

Les alkylglycosides ou alkylpolyglycosides (APG) sont des composés tensioactifs non ioniques bien connus qui peuvent être utilisés seuls, ou en association avec d'autres tensioactifs, dans une large gamme d'applications industrielles et notamment dans le domaine cosmétique.

Les alkylpolyglycosides ont d'abord été utilisés comme agents moussants et dans cette application, ceux dont la chaîne alkyle comporte de 8 à 14 atomes de carbone se sont avérés particulièrement intéressants.

Plus récemment, les alkylpolyglycosides ont été utilisés comme émulsionnants, et dans cette application, ceux dont la chaîne alkyle comporte de 16 à 18 atomes de carbone se sont avérés particulièrement intéressants.

La demande de brevet WO 92/06778, au nom de la demanderesse, décrit pour la première fois l'utilisation de mélanges d'alkylpolyglycosides et d'alcools gras en tant qu'agents auto-émulsionnants.

Par "auto-émulsionnant", on désigne tout agent ou composition capable de former une émulsion stable avec une phase aqueuse, pratiquement sans apport d'énergie, par exemple par dispersion dans la phase aqueuse par agitation mécanique lente.

Plus précisément, les mélanges décrits dans ce document antérieur comprennent :
- de 60 à 90% en poids d'au moins un alcool gras ayant de 12 à 22 atomes de carbone, et de préférence de 16 à 18 atomes de carbone ; et
- de 10 à 40 % en poids d'un alkylpolyglycoside, dont la partie alkyle est de préférence identique à celle de l'alcool gras.

Les compositions auto-émulsionnables décrites dans la demande précitée sont commercialisées sous la dénomination Montanov® 68 et comportent un mélange d'alkylpolyglycosides dont les chaînes grasses comprennent 16 et 18 atomes de carbone, ainsi qu'un mélange d'alcools gras de même longueur de chaînes grasses.

Par ailleurs, la demande de brevet WO 95/13863, au nom de la demanderesse, décrit des compositions également à base d'alkylpolyglycosides et d'alcools gras, se présentant sous forme de concentrés, notamment utiles pour la préparation d'émulsions fluides.

Ces compositions sont essentiellement caractérisées par le fait qu'elles comprennent un mélange d'au moins deux alkylpolyglycosides se différenciant par la nature de leur partie alkyle.

Il est précisé que l'un au moins de ces alkylpolyglycosides comporte une chaîne alkyle ayant de 16 à 22 atomes de carbone, et de préférence de 16 à 18 atomes de carbone.

Il est en outre précisé que les alkylpolyglycosides comportant une chaîne alkyle ayant de 16 à 22 atomes de carbone doivent représenter 25 % au moins du mélange d'alkylpolyglycosides, et dans tous les exemples, ces alkylpolyglycosides comportent 16 ou 18 atomes de carbone et représentent au moins 50 % en poids du mélange d'alkylpolyglycosides.

Il a été constaté que les compositions à base d'alkylpolyglycosides et d'alcools gras de l'état de la technique ne permettent pas d'obtenir des émulsions qui soient simultanément stables et opaques, notamment lorsqu'elles sont utilisées pour la préparation de formulations moussantes à base de tensio-actifs anioniques.

Dans ces conditions, la présente invention a pour but de résoudre le problème technique consistant en la fourniture de nouvelles compositions permettant la préparation d'émulsions dont les propriétés d'opacité sont significativement améliorées par rapport à celles des émulsions obtenues à partir des compositions décrites dans l'état de la technique dont le contenu a été rappelé précédemment, sans perte notable de stabilité.

La solution conforme à la présente invention pour résoudre ce problème technique consiste en de nouvelles compositions à base d'alkylpolyglycosides et de diols gras, caractérisées en ce qu'elles comprennent :
- 5 à 95 parties en poids d'un mélange d'alkylpolyglycosides constitué des produits de réaction d'un saccharide et du 1, 12-octadécanediol ;
- 95 à 5 parties en poids de 1, 12-octadécanediol.

De telles compositions à base d'alkylpolyglycosides et de diols gras se différencient donc essentiellement des compositions de l'état de la technique par le fait qu'elles comportent obligatoirement des alkylpolyglycosides dérivés du 1, 12-octadécanediol et que le monoalcool est remplacé par un diol.

Il a été découvert, de façon tout à fait surprenante et inattendue, que de telles compositions permettent d'obtenir des émulsions présentant des propriétés d'opacité et de stabilité remarquables, particulièrement intéressantes, pour leurs utilisations dans le domaine cosmétique, en particulier au sein de formulations moussantes.

Les compositions préférées dans le cadre de la présente invention sont les compositions comprenant :
- 5 à 60 parties en poids du mélange précité d'alkylpolyglycosides ; et
- 95 à 40 parties en poids de 1, 12-octadécanediol.

Le mélange d'alkylpolyglycosides constitué du produit de réaction d'un saccharide et du 1, 12-octadécanediol est en fait constitué d'un mélange en toutes proportions d'hydroxyoctadécylpolyglycosides (produit résultant de l'acétalisation de l'un des deux groupes hydroxyles du diol, de préférence le groupe présent en position 1), et de polyglycosyloctadécylpolyglycosides (produit résultant de l'étherification des deux groupes hydroxyles du diol).

Ces alkylpolyglycosides peuvent être représentés, respectivement par les formules I et II suivantes :

HO-R-O(G)ₙ (I)

(G)ₘ-OR-O-(G)ₚ (II)

dans lesquelles :
G représente un reste de saccharide ;
R représente un groupe octadécane 1, 12-disubstitué ;
n, m et p représentent le degré de polymérisation moyen de chaque reste de saccharide.

Les alkylpolyglycosides précités peuvent comporter, à titre de reste de saccharide, un reste de glucose ou dextrose, fructose, galactose, mannose, ribose, xylose, de préférence un reste de glucose.

Il est en outre à noter que chaque unité de la partie polyoside des alkylpolyglycosides précités peut être sous forme anomérique α ou β, et le reste de saccharide peut être de type furanoside ou pyranoside.

Le degré de polymérisation moyen de chaque reste de saccharide est généralement compris entre 1,05 et 2,5, de préférence encore entre 1,1 et 2.

L'expression "alkylpolyglycoside" utilisée dans le cadre de la présente demande désigne donc indifféremment un alkymonooside (degré de polymérisation égal à 1) ou un alkylpolyglycoside (degré de polymérisation supérieur à 1).

Le 1, 12-octadécanediol utilisé pour la préparation des compositions conformes à la présente invention est un diol provenant de l'hydrogénation des acides gras de l'huile de ricin.

Il est notamment commercialisé par la Société HENKEL sous la dénomination SPEZIOL® C 18/2.

Ce composé, en raison de son origine naturelle, peut contenir des proportions mineures d'impuretés. De telles impuretés peuvent être présentes en des quantités allant jusqu'à 25 % en poids du poids total de diol.

Par conséquent, les compositions conformes à la présente invention peuvent comprendre, en des proportions mineures correspondantes, de telles impuretés, ou les produits de réaction de ces impuretés avec un saccharide.

Les compositions à base d'alkylpolyglycosides et de diols gras conformes à la présente invention peuvent être préparées par simple mélange de leurs constituants en des proportions prédéterminées souhaitées.

A l'échelle industrielle, on les préparera de préférence selon l'une des deux voies classiquement utilisées pour la synthèse des alkylpolyglycosides, et par exemple par réaction, en milieu acide, entre le 1,12-octadécanediol et un saccharide disposant d'un OH anomérique, tel que le glucose ou le dextrose.

De telles voies de synthèse sont bien connues et ont été décrites dans de nombreux documents et en particulier dans les documents de la demanderesse rappelés précédemment.

Le cas échéant, cette synthèse pourra être complétée par des opérations de neutralisation, de filtration, de distillation ou d'extraction partielle du diol gras en excès ou de décoloration.

Les compositions à base alkylpolyglycosides et de diols gras conformes à la présente invention peuvent être utilisées pour la préparation d'émulsions pharmaceutiques, cosmétiques, hygiéniques ou détergentes, en particulier d'émulsions moussantes.

Parmi les émulsions moussantes qui peuvent être préparées dans le cadre de la présente invention, on citera en particulier les shampooings, les savons liquides, les gels douche, les bains moussants, les gels démaquillant visage, ainsi que les compositions détergentes telles que notamment les détergents vaisselle et les lessives liquides.

D'une façon générale, une telle composition comprendra de 1 à 25 % en poids, de préférence de 1 à 10 %, et de préférence encore de 1 à 5 % en poids de la composition à base d'alkylpolyglycosides et de diols gras conforme à la présente invention.

Ces émulsions comporteront en outre des tensio-actifs, notamment des tensio-actifs moussants.

A titre de tensio-actifs moussants, on peut citer :
- les tensio-actifs amphotères, notamment la bétaïne et ses dérivés tels les alkylbetaïnes, comme les N-alkylbétaïnes ou les C-alkylbétaïnes, les N-alkylaminobetaïnes, les alkylamidobétaïnes, les alkylphosphoamidobétaïnes ou les alkylphosphobétaïnes, la sultaïne et ses dérivés, tels les alkylamidosulfobetaïnes, les dérives de l'imidazoline, les N-alkylglycines, les N-alkyl-β-alanine, les alkylpolyaminecarboxylates et les N-alkylaminobutyrates.

Des tensio-actifs amphotères préférés sont choisis parmi la cocamido-bétaïne, la cocobetaïne, la cocoamidosultaïne, la cocoamidopropylbétaïne, la stéarylbétaïne, la stéaryl-amphocarboxylique glycinate, la cocoamidopropyl-hydroxysulfobétaïne, la cocoamphocarboxyglycinate, la coco-imidazoline carboxylate.
- les tensio-actifs non ioniques, notamment les amides de coprah, les polysorbates, les huiles de ricin hydrogénées et polyoxyéthylénées (OE) et/ou polyoxypropylénées (OP), les alkylphénols OE et/ou OP ou les alkylglycosides dont la chaîne alkyle comporte de 8 à 14 atomes de carbone, notamment ceux décrits dans la demande de brevet EP 70 074.
- les tensio-actifs cationiques, notamment ceux du type ammonium quaternaire ou les oxydes d'amine,
- et, de manière avantageuse, les tensio-actifs anioniques, notamment comme les carboxylates, les sulfonates et surtout, les sulfates. Parmi ces derniers. on préfère les alkylsulfates et les alkyléther sulfates neutralisés par un cation, tels les cations sodium, potassium, magnésium ou ammonium. Les lauryléther sulfates de sodium sont des tensio-actifs tout particulièrement préférés.

Par ailleurs, ces émulsions pourront comporter une phase huileuse.

Cette phase huileuse peut être constituée par une autre huile classiquement utilisée comme par exemple une huile d'origine végétate, naturelle, minérale ou synthétique.

D'une façon générale, les émulsions précitées comprendront jusqu'à 50%, et de préférence jusqu'à 30% en poids de phase huileuse telle que définie précédemment.

Ces émulsions peuvent être préparées par simple dispersion de la composition conforme à l'invention, et éventuellement d'une ou plusieurs huiles dans une phase hydrophile, généralement de l'eau ou un solvant hydrophile comme le glycérol ou le sorbitol.

Ainsi, selon un deuxième aspect, la présente demande vise à couvrir des émulsions comprenant au moins une phase aqueuse et une phase huileuse et, à titre d'émulsionnant, une composition à base d'alkylpolyglycosides et de diols gras telle que définie précédemment.

Les émulsions particulièrement préférés dans le cadre de la présente invention comporteront généralement :
- de 1 à 10 % en poids d'une composition à base d'alkylpolyglycosides et de diols gras tels que définis précédemment ;
- de 0 à 30 % en poids et de préférence de 2 à 30 % en poids d'au moins un tensio-actif moussant ;
- de 0 à 50 % en poids d'huile ; et
- une phase aqueuse.

L'invention sera illustrée plus en détail par les exemples suivants. donnes uniquement à titre illustratif.

### EXAMPLE 1

### Procédé de préparation d'une composition conforme à l'invention par acétalisation directe.

226,7 g de 1,12-octadécanediol (commercialisé par la Société Sidobre-Sinnova sous la dénomination SPEZIOL® C18/2) sont introduits dans un réacteur en verre de deux litres, muni d'une agitation mécanique efficace et d'une circulation d'un fluide caloporteur dans la double enveloppe et équipé d'un condenseur, d'une sonde de température.

Après fusion à 90°C du SPEZIOL® C18/2 dans le réacteur en 30 minutes, 22,5 g de dextrose anhydre sont dispersés dans le milieu réactionnel et homogénéisés à 90°C pendant 15 minutes. La température est alors fixée à 105°C, valeur à laquelle 2,5% d'acide sulfurique à 96% sont ajoutés.

Le milieu réactionnel est alors maintenu 6h00 à 105°C sous vide partiel.

Après refroidissement à 90°C, 2,6 g d'une solution de lessive de soude à 40% sont ajoutés progressivement de façon à obtenir une valeur du pH d'une solution à 5% du milieu réactionnel, comprise entre 5,5 et 7,5.

Le produit résultant est ainsi vidangé après tamisage sur un tamis de 250 µm. Le solide obtenu, de couleur blanche, présente alors un point de fusion de 74,7°C, une teneur en diol estimée à 89,9 %.

### EXEMPLE 2

### Procédé de préparation d'une composition conforme à l'invention par transétherification.

### 2a) Préparation du butylglucoside

888 g d'alcool butylique et 50 g d'heptane sont introduits dans un réacteur en verre de deux litres, équipé d'une double enveloppe, d'une agitation mécanique efficace, d'une sonde thermométrique, d'un système de distillation de type Dean-Stark.

Le milieu est porté à 85°C, température à laquelle 360 g de dextrose anhydre et 1,8 g d'acide sulfurique à 96 % sont ajoutés.

Le mélange est alors chauffé à 105-110°C pendant 4 heures avec recirculation en continu du mélange heptane/butanol qui distille.

### 2b) Transétherification

624.9 g du milieu réactionnel précédent, contenant 65 à 60% de butanol résiduel sont chauffés à 85°C et 642.2 g de 1,12-octadécanediol (SPEZIOL® C18/2) sont ajoutés en dispersion.

Le mélange est alors chauffé à 95°C, sous vide partiel, pendant 5 heures avec distillation continue de l'alcool butylique.

Le milieu réactionnel est ensuite refroidi à 85°C, neutralisé de façon à obtenir une valeur de pH d'une solution à 5% du milieu réactionnel comprise entre 5,5 et 7,5 avec une solution de lessive de soude.

Le produit obtenu contient alors 50 % de diol.

### EXEMPLE 3

### Procédé de préparation d'une composition conforme à l'invention par acétalisation directe

816,3 g de 1,12-octadécanediol (commercialisé par la société Sidobre-Sinnova sous la dénomination Speziol® C_{18/2}) sont introduits dans un réacteur en verre de deux litres, muni d'une agitation mécanique efficace et d'une circulation d'un fluide caloporteur dans la double enveloppe et équipé d'un condenseur et d'une sonde de température.

Après fusion à 95°C du Speziol® C_{18/2}, 198.0 g de xylose sont dispersés progressivement dans le milieu réactionnel et homogénéisés à 95°C pendant une heure.

3,03 g d'acide sulfurique à 96 % et 2,01 g d'acide hypophosphoreux à 50 % sont ajoutés et le mélange réactionnel est maintenu à 95°C pendant 4 heures sous vide partiel, avec un barbotage d'azote.

Après refroidissement à 80°C, le mélange est neutralisé de façon à atteindre une valeur du pH d'une solution du milieu réactionnel d'environ 7,2 par ajout d'une solution de borohydrure de sodium dans la lessive de soude.

Le produit résultant est ainsi vidange après tamisage sur un tamis de 250 µm. Le solide obtenu, de couleur beige, présente alors une teneur en diol estimée à 55 %.

### EXEMPLE 4

### Mise en évidence des propriétés opacifiantes des émulsions obtenues par la mise en oeuvre des compositions selon l'invention, par comparaison aux compositions de l'état de la technique

On a préparé diverses émulsions au moyen des compositions des exemples 1 et 2 selon l'invention ainsi que des compositions de l'état de la technique.

Ces émulsions ont été préparées de la façon suivante :

Les compositions à base d'APG et d'alcools gras sont fondus à chaud dans une solution aqueuse de tensio-actifs anioniques. L'ensemble est homogénéisé sous agitation et dilué dans l'eau restante puis refroidi.

En suivant ce protocole expérimental, on a préparé diverses émulsions à partir des compositions selon l'invention décrites aux exemples 1 et 2 et à partir des compositions de comparaison décrites respectivement aux exemples 1 du document WO 92/06778 (exemple comparatif 1) et 1 du document WO 95 13863 (exemple comparatif 2).

Ces émulsions présentent les compositions suivantes :
- compositions selon l'invention
   ou selon un exemple comparatif : 5%.
- tensio-actif anionique (LESNA) : 10%.
- eau : 85%.

La stabilité des émulsions ainsi préparées est contrôlée après vieillissement à 40°C.

L'opacité est mesurée à l'aide d'un chromamètre MINOLTA CR 200 équipé d'un cône et d'un tube de protection CR-A33a.

Les mesures sont effectuées en immergeant le cône dans 50 ml d'émulsion.

L'opacité est décrite par le paramètre L, variation de 0 à 100.

Les résultats obtenus ont été reportés au tableau I ci-après :

Dans ce tableau :
Dph signifie : déphase au bout de
J signifie : jours

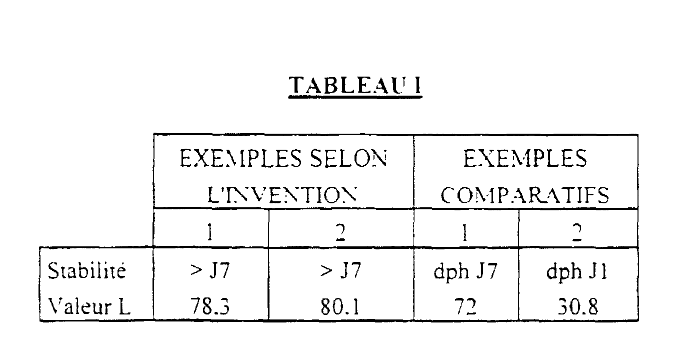

Comme le montre le tableau I. les émulsions obtenues à partir des compositions de l'invention présentent un effet opacifiant significativement superieur à celui des émulsions obtenues à partir des compositions de l'état de la technique, avec augmentation de stabilité.

### EXEMPLE 5

### Exemples d'émulsions incorporant les compositions à base d'alkylpolyglycosides et de diols gras selon l'invention

| **SAVON LIQUIDE** | | |
|---|---|---|
| A) | Composition de l'Exemple 1 | 4% |
| | Eau | 30% |
| | | |
| B) | Les Na | 5% |
| | ORAMIX® NS 10 | 3% |
| | Eau | Qs |
| | | |
| C) | CAPIGEL® 98 | 2% |
| | Eau | Qs |
| | | |
| D) | Parfum | Qs |
| | Conservateur | Qs |

La composition de l'exemple 1 est fondue dans l'eau à environ 80°C (phase A) sous agitation. Après refroidissement vers 30°C, on ajoute les phases B, C et D. On ajuste le pH vers 7 avec de la soude.

| **LAIT DEMAQUILLANT MOUSSANT** | | |
|---|---|---|
| A) | Composition de l'Exemple 2 | 4% |
| | Huile de jojoba | 4% |
| | | |
| B) | Eau | Qs |
| | CAPIGEL® 98 | 3 % |
| | | |
| C) | Parfum conservateur | Qs |
| | LESNA | 7% |

On fond la phase A vers 80°C. On ajoute la phase B sous agitation à la phase A ainsi fondue. On refroidit vers 30°C puis on ajoute la phase C et on ajuste le pH vers 7.

| **LAIT DEMAQUILLANT** | | |
|---|---|---|
| A) | Composition de l'Exemple 2 | 4% |
| | Huile de paraffine | 15 % |
| | | |
| B) | Eau | |
| | | |
| C) | SEPIGEL® 305 | 1 % |
| | | |
| D) | Parfum, conservateur | |

On fond la composition de l'exemple 2 dans l'huile vers 80°C. On ajoute la phase B sous agitation à la phase A ainsi fondue. On ajoute la phase C vers 60°C, puis on ajoute la phase D vers 30°C.

## Revendications

1. Compositions à base d'alkylpolyglycosides et de diols gras, **caractérisées en ce qu'**elles comprennent :
- 5 à 95 parties en poids d'un mélange d'alkylpolyglycosides constitué des produits de reaction d'un saccharide et du 1,12-octadécanediol ;
- 95 à 5 parties en poids de 1,12-octadécanediol.

2. Compositions selon la revendication 1, **caractérisées en ce qu'**elles comprennent :
- 5 à 60 parties en poids du mélange précité d'alkylpolyglycosides ; et
- 95 à 40 parties en poids de 1,12-octadécanediol.

3. Compositions selon l'une des revendications 1 ou 2, **caractérisées en ce que** le mélange d'alkylpolyglycosides précité est constitué d'un mélange en toutes proportions d'hydroxyoctadécylpolyglycosides de formule (I) et de polyglycosyloctadecylpolyglycosides de formule (II)
HO-R-O(G)ₙ (I)
(G)ₘ-OR-O-(G)ₚ (II)
dans lesquelles :
G représente un reste de saccharide ;
R représente un groupe octadécane 1, 12-disubstitué ;
n, m et p représentent le degré de polymérisation moyen de chaque reste de saccharide.

4. Compositions selon la revendication 3, **caractérisées en ce que** dans les formules (I) et (II) précitées :
- G représente un reste de glycose choisi parmi le glucose, dextrose, fructose, galactose, mannose, ribose ou xylose ; et
- n, m et p représentent un nombre compris entre 1,05 et 2,5, de préférence entre 1,1 et 2.

5. Emulsions du type comprenant une phase aqueuse, une phase huileuse et un émulsionnant, **caractérisées en ce que** ledit émulsionnant est constitué par une composition à base d'alkylpolyglycosides et de diols gras tels que définis selon l'une quelconque des revendications 1 à 4.

6. Emulsions selon la revendication 5, **caractérisées en ce qu'**elles comprennent :
- de 1 à 10% en poids d'une composition à base d'alkylpolyglycosides et de diols gras tels que définis précédemment ;
- de 0 à 30 % en poids et de préférence de 2 à 30% en poids d'au moins un tensio-actif moussant ;
- de 0 à 50% en poids d'huile ; et
- une phase aqueuse.

## Patentansprüche

1. Zusammensetzungen auf der Basis von Alkylpolyglycosiden und Fettdiolen, **dadurch gekennzeichnet, dass** sie
- 5 bis 95 Gewichtsteile eines Gemischs von Alkylpolyglycosiden, bestehend aus Reaktionsprodukten von einem Saccharid und von 1,12-Octadecandiol; und
- 95 bis 5 Gewichtsteile 1,12-Octadecandiol
umfassen.

2. Zusammensetzungen gemäß Anspruch 1, **dadurch gekennzeichnet, dass** sie
- 5 bis 60 Gewichtsteile des vorstehenden Gemischs von Alkylpolyglycosiden; und
- 95 bis 40 Gewichtsteile 1,12-Octadecandiol
umfassen.

3. Zusammensetzungen gemäß einem der Ansprüche 1 oder 2, **dadurch gekennzeichnet, dass** das vorstehende Gemisch von Alkylpolyglycosiden aus einem Gemisch eines beliebigen Verhältnisses von Hydroxyoctadecylpolyglycosiden der Formel (I) und Polyglycosyloctadecylpolyglycosiden der Formel (II) besteht
HO-R-O(G)ₙ (I)
(G)ₘ-OR-O-(G)ₚ (II)
wobei:
G einen Saccharidrest darstellt;
R eine 1,12-disubstituierte Octadecangruppe darstellt; und
n, m und p den mittleren Polymerisationsgrad des jeweiligen Saccharidrestes darstellen.

4. Zusammensetzungen gemäß Anspruch 3, **dadurch gekennzeichnet, dass** in den vorstehenden Formeln (I) und (H):
G einen Glucoserest, ausgewählt aus Glucose, Dextrose, Fructose, Galactose, Mannose, Ribose oder Xylose darstellt; und
n, m und p eine Zahl zwischen 1,05 und 2,5, vorzugsweise zwischen 1,1 und 2 darstellen.

5. Emulsionen, die eine wässrige Phase, eine ölige Phase und einen Emulgator umfassen, **dadurch gekennzeichnet, dass** der Emulgator aus einer Zusammensetzung auf der Basis von Alkylpolyglycosiden und Fettdiolen besteht, wie in einem der Ansprüche 1 bis 4 definiert.

6. Emulsionen gemäß Anspruch 5, **dadurch gekennzeichnet, dass** sie
- 1 bis 10 Gewichtsprozent einer Zusammensetzung auf der Basis von Alkylpolyglycosiden und Fettdiolen, wie vorstehend definiert;
- 0 bis 30 Gewichtsprozent und vorzugsweise 2 bis 30 Gewichtsprozent mindestens eines schaumbildenden oberflächenaktiven Mittels;
- 0 bis 50 Gewichtsprozent Öl; und
- eine wässrige Phase
umfassen.

## Claims

1. Compositions based on alkylpolyglycosides and fatty diols, **characterised in that** they comprise :
- 5 to 95 parts by weight of a mixture of alkylpolyglycosides which is constituted of the products of a reaction of a saccharide and 1,12-octadecanediol ;
- 95 to 5 parts by weight of 1,12-octadecanediol.

2. Compositions according to claim 1, **characterised in that** they comprise :
- 5 to 60 parts by weight of the above-mentioned mixture of alkylpolyglycosides ; and
- 95 to 40 parts by weight of 1,12-octadecanediol.

3. Compositions according to one of claims 1 or 2, **characterised in that** the mixture of alkylpolyglycosides mentioned above is constituted of a mixture of any proportion of hydroxyoctadecylpolyglycosides of formula (I) and of polyglycosyloctadecylpolyglycosides of formula (II)
HO-R-O(G)ₙ (I)
(G)ₘ-OR-O-(G)ₚ (II)
in which:
G represents a saccharide residue ;
R represents a 1,12-disubstituted octadecane group;
n, m and p represent the average degree of polymerisation of each saccharide residue.

4. Compositions according to claim 3, **characterised in that** in the formulae (I) and (II) mentioned above :
- G represents a glycose residue selected from glucose, dextrose, fructose, galactose, mannose, ribose or xylose ; and
- n, m and p represent a number between 1.05 and 2.5, preferably between 1.1 and 2.

5. Emulsions of the type comprising an aqueous phase, an oily phase and an emulsifier, **characterised in that** said emulsifier is constituted by a composition based on alkylpolyglycosides and fatty diols as defined according to any one of claims 1 to 4.

6. Emulsions according to claim 5, **characterised in that** they comprise:
- from 1 to 10 % by weight of a composition based on alkylpolyglycosides and fatty diols as defined above ;
- from 0 to 30 % by weight and preferably from 2 to 30 % by weight of at least one foaming surfactant ;
- from 0 to 50 % by weight of oil; and
- an aqueous phase.
